# EUROPÄISCHE PATENTSCHRIFT

(11) **EP 3 713 641 B1**
(45) Veröffentlichungstag und Bekanntmachung des Hinweises auf die Patenterteilung: **05.01.2022**
(21) Anmeldenummer: 18808327.3
(22) Anmeldetag: 23.11.2018
(51) Int. Cl.: A61N 2/02

(54) **MAGNETFELDGENERATOR ALS TRAGBARER NATURFELDSIMULATOR**
MAGNETIC FIELD GENERATOR AS A PORTABLE NATURAL FIELD SIMULATOR
GÉNÉRATEUR DE CHAMP MAGNÉTIQUE EN TANT QUE SIMULATEUR PORTABLE DE CHAMP NATUREL

(30) Priorität: 24.11.2017 DE 102017010886; 27.11.2017 DE 102017010926
(43) Veröffentlichungstag der Anmeldung: 30.09.2020
(73) Patentinhaber: Feucht, Peter, 12247 Berlin (DE)
(72) Erfinder: Feucht, Peter, 12247 Berlin (DE)
(74) Vertreter: Liebl, Thomas
(86) Internationale Anmeldenummer: PCT/EP2018/082405
(87) Internationale Veröffentlichungsnummer: WO 2019/101941

(56) Entgegenhaltungen:
- DE-A1-102015 011 092
- ES-A1- 2 247 895
- ES-B1- 2 247 895
- US-A1- 2016 158 541
- Dieter Broers: "Naturfeldstabilisator NFS 8", , 16. Oktober 2017 (2017-10-16), XP055567814, Gefunden im Internet: URL:https://web.archive.org/web/2017101612 2544/https://dieter-broers.de/naturfeldsta bilisator-nfs-8/ [gefunden am 2019-03-12]

## Beschreibung

Die Erfindung betrifft einen Magnetfeldgenerator als tragbaren Naturfeldsimulator nach dem Oberbegriff des Anspruchs 1.

Ein Magnetfeldgenerator als Naturfeldsimulator in einem am Körper tragbaren Gehäuse in dem eine Feldspule eine Elektronik und eine Batterie enthalten sind, ist aus DE 10 2015 011 092 A1 bekannt. In diesem Dokument sind Maßnahmen zur Miniaturisierung des Magnetfeldgenerators dargestellt.

Anmeldungsgemäß werden zwei wesentliche Natur-Wechsel-Felder als Magnetwechselfelder gleichzeitig simuliert:
Als erstes Natur-Wechsel-Feld werden sogenannte "Schumann-Resonanzen" simuliert, die 1955 von dem Physiker Winfried Otto Schumann nachgewiesen wurden (siehe unter Sferics-Wikipedia). Der Raum zwischen der Erde und der Ionosphäre kann als Hohlraumresonator wirken. Schumann-Resonanzen sind diejenigen Frequenzen, bei denen die Wellenlänge elektromagnetischer Schwingungen im Hohlleiter zwischen Erdoberfläche und Ionosphäre ein ganzzahliger Teil des Erdumfangs ist. Die Energie für die Anregung solcher Schwingungen stammt aus der weltweiten Gewittertätigkeit. Die Grundwelle der Schumann-Resonanzen liegt rechnerisch genau bei 7,83 Hz, wobei dieser Wert jedoch insbesondere durch Überlagerungen unscharf ist und auch tageszeitabhängig in einer Bandbreite um 7,83 Hz schwanken kann.

Als zweite, gleichzeitig mit den "Schumann-Resonanzen" auftretende Natur-Wechsel-Felder werden sogenannte Wetter-Sferics, insbesondere sogenannte Schönwetter-Sferics simuliert, welche bereits zu Beginn des 20. Jahrhunderts entdeckt worden sind und deren Wirkungen gezielt seit etwa 1980 untersucht worden sind. Solche Sferics sind impulsartige, gedämpfte Schwingungen mit Frequenzen zwischen 3 und 100 KHz bei einer Impulsfolgefrequenz von maximal 150 Hz. Anmeldungsgemäß werden typische Schönwetter-Sferics mit einer Resonanzfrequenz von 5 bis 30 KHz, vorzugsweise von 10 KHz mit einer Impulsfolgefrequenz entsprechend der simulierten Schumann-Resonanzen simuliert.

Die vorstehend erläuterten Natur-Wechsel-Felder als Magnetwechselfelder sind erdgeschichtlich ständig vorhanden, so dass sich auf deren Einfluss und Wirkung die gesamte Schöpfung und Evolution eingestellt hat. Chemisch/Physikalische Wirkungen sind experimentell nachgewiesen. Untersuchungen zu biologischen Wirkungen, insbesondere zu biologisch positiven Wirkung auf den Menschen wurden und werden durchgeführt.

In Ballungsgebieten der Erde, insbesondere in Hochhäusern unserer Städte sind die vorstehenden Natur-Wechsel-Felder oft nur sehr abgeschwächt vorhanden. Während das statische geomagnetische Feld solche Bauten weitgehend durchdringen und Stahlbauten zu polarisieren vermag, werden die Magnetwechselfelder sehr stark abgeschwächt und zudem teilweise völlig durch Störfelder elektrischer Anlagen und Sender aller Art verdeckt. Somit sind unter solchen Bedingungen praktisch keine experimentellen Untersuchungen zu natürlichen Schumann-Resonanzen und Sferics durchführbar oder Wirkungen auf den Menschen feststellbar.

Aufgabe der Erfindung ist es daher, die eingangs erläuterten Natur-Wechsel-Felder als Magnetwechselfelder mit typischen Werten nachzubilden und zu simulieren und damit für unterschiedliche Zwecke, insbesondere auch für eine Beeinflussung des menschlichen Körpers zur Verfügung zu stellen und nutzbar zu machen.

Diese Aufgabe wird mit den Merkmalen des Anspruchs 1 gelöst.

Gemäß Anspruch 1 weist der Magnetfeldgenerator als tragbarer Naturfeldsimulator eine elektrische Schaltung mit einem Stromkreis auf, in dem in Reihe eine Batterie, eine mit dieser verbundene Feldspule sowie ein dieser nachgeschalteter Schalttransistor angeordnet sind. Die Basis des Schalttransistors ist mit einem Taktgeber für einen Takt von 6 Hz bis 10 HZ, vorzugsweise von 8 Hz, zur Erzeugung entsprechender Rechteckimpulse verbunden. Dadurch ist die Feldspule als Induktivität im Takt des Taktgebers an der Batteriespannung ein- und ausschaltbar, wodurch ein Magnet-Wechselfeld mit einer Schwingung von 6 bis 10 Hz, vorzugsweise von 8 Hz, zur naturnahen Simulation der Grundfrequenz der "Schumann-Resonanzen" erzeugbar ist.

Weiter ist ein Kondensator in einer Parallelschaltung zur Feldspule zur Ausbildung eines Schwingkreises mit einer Resonanzfrequenz von 5 bis 30 KHz, vorzugsweise von 10 KHz, angeordnet.

Damit wären jeweils nach dem getakteten Ausschalten der Feldspule mit der simulierten Grundfrequenz der Schumann-Resonanzen getaktete, gedämpft abklingende elektromagnetische Schwingungen von 5 bis 30 KHz, vorzugsweise von 10 KHz, zur naturnahen Simulation von Sferics-Impulsen als sogenannte "Schönwetter-Sferics" erzeugbar.

Der Nachteil ist allerdings, dass mit dieser Schaltung aktiv nur eine positive 10 KHz-Halbwelle generiert werden kann, denn sobald die Schwingung der Kollektorspannung negative Werte annimmt, wird die negative KollektorSpannung bei etwa -0,5 Volt infolge Durchbruchs des Transistors begrenzt und dem Schwingkreis die Energie entzogen, so dass keine weiteren Schwingungen mehr möglich sind.

Erfindungsgemäß wird durch das Einfügen der Diode (D1) der Energieentzug des Schwingkreises verhindert. Damit wird nach der Abschaltung des Transistors dem Schwingkreis aus Feldspule und Kondensator das freie Ausschwingen mit einer gedämpften Schwingung ermöglicht.

Die Diode (D1) kann zwischen Batterie (+V_{B}) und Schwingkreis (S) und/oder zwischen dem Schwingkreis (S) und dem Schalttransistor (T) angeordnet werden. In dieser zweiten Anordnung können der Schalttransistor (T) und die Diode (D1) in einem Bauelement integriert sein. Gegebenenfalls kann auch ein Bauelement mit entsprechender Bauart für die hier erforderlichen Spannungen ohne störenden Rückstrom vorgesehen sein.

Zum Schutz des Transistors und zur Vermeidung von Verlusten ist es weiter zweckmäßig und vorteilhaft, eine zweite Diode (D2) parallel zum Transistor zu schalten.

In einer konkreten bevorzugten Ausführungsform soll die Feldspule eine Induktivität von 7 bis 8 Henry, vorzugsweise von 7,68 Henry, aufweisen. Zudem soll der Kondensator eine Kapazität von 30 bis 40 pF, vorzugsweise von 33 pF, aufweisen (mit 1 pF = 1 * 10 exp -12 Farad).

Für eine zur Beeinflussung eines Menschen bestimmte Ausführungsform eines Naturfeldsimulators soll ein am Körper eines Menschen tragbares Gehäuse aufweisen, in dem die elektrische Schaltung enthalten ist.

In einem solchen Gehäuse kann weiter ein Permanentmagnet enthalten sein, mit dem auch das statische Erdmagnetfeld simulierbar ist und dessen Feldstärke in einem geringen Abstand vom Gehäuse, vorzugsweise von ca. 3 cm dem natürlichen statischen Erdmagnetfeld entspricht.

Anhand einer Zeichnung wird die Erfindung beispielhaft anhand eines von einem Menschen tragbaren Naturfeldsimulators näher erläutert.

Es zeigen:
- Figur 1: eine Draufsicht auf einen Magnetfeldgenerator als Naturfeldsimulator,
- Figur 2: eine Seitenansicht des Naturfeldsimulators nach Figur 1,
- Figur 3: eine Draufsicht auf die interne Baugruppe des Naturfeldsimulators nach Figur 1, und
- Figur 4: eine im Naturfeldsimulator enthaltene Schaltung zur Erzeugung der Grundschwingung der Schumann-Resonanzen und zur Erzeugung von magnetischen Sferics-Impulsen.

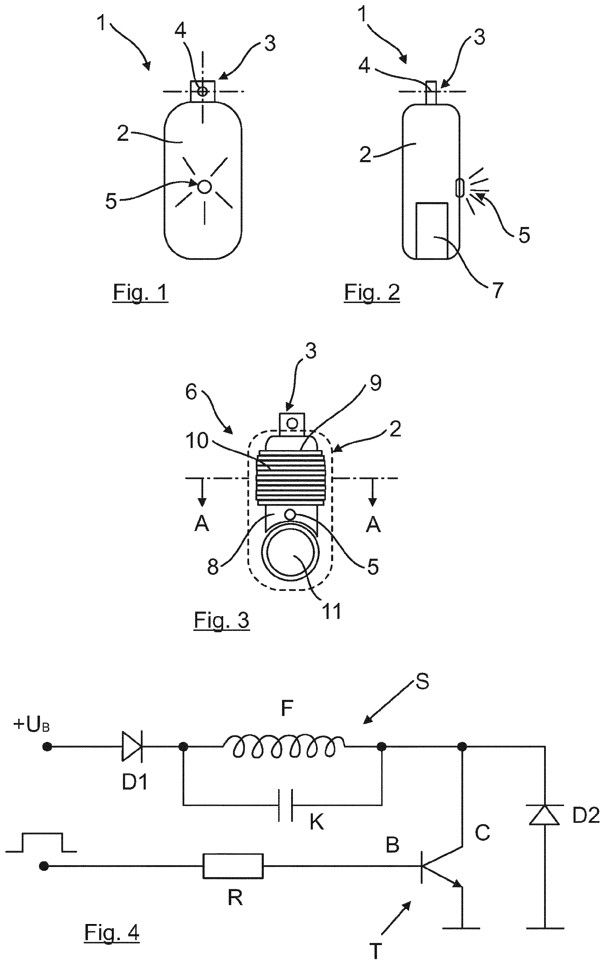

In den Figuren 1 und 2 sind eine Draufsicht und eine Seitenansicht eines Magnetfeldgenerators als Naturfeldsimulator 1 etwa in natürlicher Größe dargestellt. Aus dem Gehäuse 2 ragt an der Oberseite ein Drehschalter 3, der zugleich mit einer Öse 4 als Aufhänger verwendbar ist, so dass der Naturfeldsimulator 1 an einem Halsband getragen werden kann.

An der Frontseite des Naturfeldsimulators 1 ist eine mit einem Kristall abgedeckte Lichtaustrittöffnung angebracht, hinter der eine Leuchtdiode 5 einer Baugruppe 6 liegt (Figur 3).

Zudem weist das Gehäuse 2 eine durch eine Verschlusskappe 7 lösbar abgedeckte Öffnung als Batteriefach auf, durch die auch die Baugruppe 6 bei der Montage eingeführt und fixiert werden kann.

Die Baugruppe 6 besteht im Wesentlichen aus einer Elektronikplatine 8, einem Magnet-Eisenkern als Eisenkern-Blechrohr 9, einer Magnetspule 10, die auf das Eisenkern-Blechrohr 9 gewickelt ist, und aus einer Lithium-Knopfbatterie 11. Die Baugruppe 6 ist im montierten Zustand in das Gehäuse 2 eingesteckt und füllt dieses weitgehend aus, wie dies mit der strichlierten Linie als Gehäuse 2 angedeutet ist.

Die zum Betrieb des Naturfeldsimulators 1 verwendete bevorzugte Schaltung ist in Figur 4 dargestellt und weist einen Schwingkreis S auf, der aus einer Feldspule F (entsprechend der Magnetspule 10) und einem parallel geschalteten Kondensator K gebildet ist mit einer Resonanzfrequenz von hier beispielsweise und bevorzugt 10 KHz als charakteristischer Frequenz von "Schönwetter-Sferics".

Zwischen dem Pluspol +U_{B} einer (hier nicht dargestellten) Batterie und dem nachgeschalteten Schwingkreis S ist eine Diode D1 im Stromfluss geschaltet.

Anschließend an den Schwingkreis S ist ein Transistor T mit seinem Kollektor C verbunden. Die Diode D1 könnte alternativ auch zwischen dem Schwingkreis S und dem Transistor T angeordnet sein. Die Basis B des Transistors T ist über einen Widerstand R mit einem (nicht explizit dargestellten) Rechteckgenerator als Taktgeber für hier beispielweise und bevorzugt 8 Hz verbunden. Zudem ist hier beispielhaft eine weitere Diode D2 entgegengerichtet zur ersten Diode D1 parallel zum Transistor T geschaltet.

Die dargestellte Schaltung hat folgende Funktion:
Zur Simulation der Grundwelle der Schumann-Resonanzen wird mit dem Rechteckgenerator die Basis B des Schaltgenerators T so angesteuert, dass der Stromkreis über die Feldspule F in einem charakteristischen Takt von hier beispielsweise und bevorzugt 8 Hz ein- und ausgeschaltet wird.

Zugleich werden Sferics-Impulse in der Art von Schönwetter-Sferics erzeugt und simuliert mit einer charakteristisch gewählten Frequenz von hier beispielsweise und bevorzugt 10 KHz und einer charakteristischen Impulsfolgefrequenz von hier beispielsweise und bevorzugt 8 Hz entsprechend der erzeugten und simulierten Grundfrequenz der Schumann-Resonanzen. Dazu wird im hier gezeigten Beispielfall jeweils nach dem getakteten Ausschalten der Feldspule F aus dem Abbau des Magnetfelds der Feldspule F eine Periode von gedämpft abklingenden elektromagnetischen 10 KHz-Schwingungen erzeugt, so dass damit eine naturnahe Simulation von 10 KHz-Sferics-Impulsen erzeugt werden kann. Mit der Diode D1 wird dazu das freie Ausschwingen des Schwingkreises S mit einer gedämpften Schwingung ermöglicht. Die weitere, optionale Diode D2 dient als Schutz des Transistors T vor negativen Spannungen am Kollektor C des Transistors T.

## Patentansprüche

1. Magnetfeldgenerator, ausgebildet als tragbarer Naturfeldsimulator, mit einer elektrischen Schaltung mit einem Stromkreis, in dem in Reihe eine Batterie (+U_{B}), eine mit dieser verbundene Feldspule (F) sowie ein dieser nachgeschalteter Schalttransistor (T) angeordnet sind, dessen Basis (B) mit einem Taktgeber für einen Takt von 6 Hz bis 10 Hz, vorzugsweise von 8 Hz, verbunden ist, so dass die Feldspule (F) als Induktivität im Takt des Taktgebers an der Batteriespannung (+U_{B}) ein- und ausschaltbar ist, wodurch ein Magnet-Wechselfeld mit einer Schwingung von 6 Hz bis 10 Hz, vorzugsweise von 8 Hz, zur naturnahen Simulation der Grundfrequenz der Schumann-Resonanzen erzeugbar ist,
**dadurch gekennzeichnet,**
**dass** ein Kondensator (K) in einer Parallelschaltung zur Feldspule (F) zur Ausbildung eines Schwingkreises (S) mit einer Resonanzfrequenz von 5 bis 30 KHz, vorzugsweise von 10 KHz, angeordnet ist und zudem im Stromfluss zwischen Batterie (+U_{B}) und Schwingkreis (S) und/oder zwischen dem Schwingkreis (S) und dem Schalttransistor (T) eine Diode (D1) geschaltet ist,
wodurch jeweils nach dem getakteten Ausschalten der Feldspule (F) aus dem Abbau des Magnetfelds der Feldspule (F) mit der Frequenz der simulierten Grundfrequenz der Schumann-Resonanzen getaktete,
gedämpft abklingende elektromagnetische Schwingungen von 5 bis 30 KHz, vorzugsweise von 10 KHz, zur naturnahen Simulation von Sferics-Impulsen als Schönwetter-Sferics erzeugbar sind.

2. Magnetfeldgenerator nach Anspruch 1, enthaltend ein Bauelement, in welches der Schalttransistor (T) und die Diode (D1) nach dem Schwingkreis integriert sind oder welches an dieser Stelle für die hier erforderlichen negativen Spannungen ohne störenden Rückstrom vorgesehen ist.

3. Magnetfeldgenerator nach Anspruch 1 oder 2, wobei parallel zum Transistor (T) entgegengerichtet zur ersten Diode (D1) eine zweite Diode (D2) geschaltet ist.

4. Magnetfeldgenerator nach Anspruch 1 oder Anspruch 3, wobei die Feldspule (F) eine Induktivität von 7 bis 8 Henry, vorzugsweise von 7,68 Henry, aufweist, und
dass der Kondensator (K) eine Kapazität von 30 bis 40 pF, vorzugsweise von 33 pF, aufweist.

5. Magnetfeldgenerator nach einem der Ansprüche 1 bis 4, wobei
der Naturfeldsimulator (1) ein am Körper eines Menschen tragbares Gehäuse (2) aufweist, in dem die elektrische Schaltung enthalten ist.

6. Magnetfeldgenerator nach Anspruch 5, wobei
in dem Gehäuse (2) weiter ein Permanentmagnet enthalten ist, mit dem das statische Erdmagnetfeld simulierbar ist, dessen Feldstärke in einem geringen Abstand vom Gehäuse (2), vorzugsweise von ca. 3 cm, dem natürlichen, statischen Erdmagnetfeld entspricht.

## Claims

1. Magnetic field generator, designed as a portable natural field simulator, comprising an electrical circuit having a circuit in which a battery (+U_{B}), a field coil (F) connected thereto and a downstream switching transistor (T) are arranged in series, the base (B) of the switching transistor being connected to a clock generator for a clock rate of 6 Hz to 10 Hz, preferably of 8 Hz, so that the field coil (F) can be switched on and off as an inductance at the battery voltage (+U_{B}) at the clock rate of the clock generator, as a result of which an alternating magnetic field having an oscillation of 6 Hz to 10 Hz, preferably of 8 Hz, can be generated for near-natural simulation of the fundamental frequency of the Schumann resonances,
**characterized in that**
a capacitor (K) is arranged in a parallel circuit to the field coil (F) in order to form a resonant circuit (S) having a resonance frequency of 5 to 30 kHz, preferably of 10 kHz and, furthermore, a diode (D1) is connected in the current flow between the battery (+U_{B}) and the resonant circuit (S) and/or between the resonant circuit (S) and the switching transistor (T),
as a result of which, after the field coil (F) has been switched off in a clocked manner, clocked electromagnetic oscillations of 5 to 30 kHz, preferably of 10 kHz, which decay in an attenuated manner, can be generated from the dissipation of the magnetic field of the field coil (F) at the frequency of the simulated fundamental frequency of the Schumann resonances, for near-natural simulation of sferics pulses as so-called fair-weather sferics.

2. Magnetic field generator according to Claim 1, containing a component in which the switching transistor (T) and the diode (D1) are integrated downstream of the resonant circuit or which is provided at this location for the negative voltages required here without a disruptive return current.

3. Magnetic field generator according to Claim 1 or 2, wherein a second diode (D2) is connected in parallel with the transistor (T) in the opposite direction to the first diode (D1).

4. Magnetic field generator according to Claim 1 or Claim 3, wherein the field coil (F) has an inductance of 7 to 8 Henry, preferably of 7.68 Henry, and the capacitor (K) has a capacitance of 30 to 40 pF, preferably of 33 pF.

5. Magnetic field generator according to one of Claims 1 to 4, wherein the natural field simulator (1) has a housing (2) which can be carried on the body of a person and which contains the electrical circuit.

6. Magnetic field generator according to Claim 5, wherein the housing (2) also contains a permanent magnet, using which the static magnetic field of the earth can be simulated, the field strength of which at a short distance from the housing (2), preferably of approximately 3 cm, corresponds to the natural static magnetic field of the earth.

## Revendications

1. Générateur de champ magnétique, réalisé sous la forme d'un simulateur de champ naturel portable, comprenant un câblage électrique pourvu d'un circuit électrique, dans lequel sont disposés en série une pile et/ou batterie (+U_{B}), une bobine inductrice (F) reliée à celle-ci ainsi qu'un transistor de commutation (T) branché à la suite de celle-ci, dont la base (B) est reliée à un générateur de rythme pour un rythme de 6 Hz à 10 Hz, de préférence de 8 Hz, de sorte que la bobine inductrice (F) peut être connectée à et déconnectée de la pile et/ou batterie (+U_{B}) en tant qu'inductance au rythme du générateur de rythme, moyennant quoi un champ magnétique alternatif ayant une oscillation de 6 Hz à 10 Hz, de préférence de 8 Hz, peut être générée pour la simulation proche de l'état naturel de la fréquence fondamentale des résonances de Schumann,
**caractérisé en ce que**
un condensateur (K) est disposé dans un circuit parallèle avec la bobine inductrice (F) en vue de former un circuit oscillant (S) ayant une fréquence de résonance de 5 à 30 kHz, de préférence de 10 kHz, et en plus de cela une diode (D1) est branchée dans le flux de courant entre la pile et/ou batterie (+U_{B}) et le circuit oscillant (S) et/ou entre le circuit oscillant (S) et le transistor de commutation (T),
moyennant quoi des oscillations électromagnétiques cadencées décroissantes atténuées de 5 à 30 kHz, de préférence de 10 kHz, peuvent être générées respectivement après la déconnexion cadencée de la bobine inductrice (F) à partir de la suppression du champ magnétique de la bobine inductrice (F) avec la fréquence de la fréquence fondamentale des résonances de Schumann en vue de la simulation proche de l'état naturel d'impulsions de parasite atmosphérique sous la forme de parasite atmosphérique de beau temps.

2. Générateur de champ magnétique selon la revendication 1, contenant un composant dans lequel le transistor de commutation (T) et la diode (D1) sont intégrés après le circuit oscillant ou qui est prévu à cet endroit pour les tensions négatives nécessaires ici sans courant de retour perturbateur.

3. Générateur de champ magnétique selon la revendication 1 ou 2, une deuxième diode (D2) étant branchée en parallèle avec le transistor (T) en tête-bêche avec la première diode (D1).

4. Générateur de champ magnétique selon la revendication 1 ou la revendication 3, la bobine inductrice (F) ayant une inductance de 7 à 8 Henry, de préférence de 7,68 Henry, et le condensateur (K) ayant une capacité de 30 à 40 pF, de préférence de 33 pF.

5. Générateur de champ magnétique selon l'une des revendications 1 à 4, le simulateur de champ naturel (1) possédant un boîtier (2) qui peut être porté sur le corps d'une personne, dans lequel est contenu le câblage électrique.

6. Générateur de champ magnétique selon la revendication 5, un aimant permanent étant en outre inclus dans le boîtier (2), avec lequel peut être simulé le champ magnétique terrestre statique, dont l'intensité de champ, à une faible distance du boîtier (2), de préférence d'environ 3 cm, correspond au champ magnétique terrestre statique naturel.
